# EUROPÄISCHE PATENTSCHRIFT

(11) **EP 1 595 567 B1**
(45) Veröffentlichungstag und Bekanntmachung des Hinweises auf die Patenterteilung: **09.09.2009**
(21) Anmeldenummer: 05009080.2
(22) Anmeldetag: 26.04.2005
(51) Int. Cl.: A61M 25/01

(54) **Katheter-Führungsdraht mit radiopaquem Marker**
Guidewire comprising a radiopaque marker for a catheter
Fil-guide à marqueur radio-opaque pour un cathéter

(30) Priorität: 10.05.2004 DE 102004023642
(43) Veröffentlichungstag der Anmeldung: 16.11.2005
(73) Patentinhaber: Biotronik VI Patent AG, 6340 Baar (CH)
(72) Erfinder: Hofmann, Eugen, 8049 Zürich (CH); Wintsch, Christoph, 8311 Brütten (CH)
(74) Vertreter: Lindner-Vogt, Karin L.

(56) Entgegenhaltungen:
- US-A- 5 209 730
- US-A- 5 479 938
- US-A1- 2002 042 582

## Beschreibung

Die Erfindung betrifft einen Katheter-Führungsdraht insbesondere für die perkutane transluminale Coronar-Angioplastik (PTCA) mit den im Oberbegriff des Anspruches 1 angegebenen Merkmalen.

Demnach weisen solche Katheter-Führungsdrähte einen langgestreckten Drahtschaft aus einem federelastischen Material mit einem proximalen und distalen Ende auf. Im distalen Endbereich sitzen eine oder mehrere radiopaque, hülsenartige Marker auf dem Drahtschaft, die im wesentlichen zwei Aufgaben besitzen. Zum einen soll - beispielsweise durch eine langgestreckte Markerwendel direkt am distalen Ende des Führungsdrahtes - die Position der Führungsdrahtspitze beim Applizieren des Katheters in einem Röntgensichtgerät gut erkennbar sein. Zum anderen kann mit Hilfe mehrerer, beispielsweise äquidistant vor dem distalen Ende angeordneter Marker eine Längen-Vermessung einer zu behandelnden Stenose auf dem Röntgensichtgerät vorgenommen werden.

Derartige Führungsdrähte sind aus einer Vielzahl von Druckschriften von ihrem grundsätzlichen Aufbau her bekannt. So zeigt die US-Patentschrift US 5,209,730 A einen Katheter-Führungsdraht, der einen langgestreckten Drahtschaft aus einem federelastischen Material, nämlich hochfestem Edelstahl aufweist. Darauf sitzen radiopaque Marker vor dem distalen Ende, die durch Schraubenfederelemente - sogenannte "coils" - gebildet sind und - wie erwähnt - zur Stenosen-Längenmessung dienen.

Die US 5,479,938 zeigt einen ähnlichen Katheter-Führungsdraht, auf dessen Schaft mit zunehmender Entfernung vom distalen Ende einen sich vergrößemden Abstand aufweisende radiopaque Marker in Form von Metallhülsen - ebenfalls zur Stenosen-Längenvermessung - sitzen. Diese können an ihren in Drahtlängsrichtung weisenden Stirnkanten angeschrägt sein.

Die US 2002/0042582 zeigt einen Katheter-Führungsdraht gemäß dem Oberbegriff des Anspruchs 1.

Bei derartigen Katheter-Führungsdrähten stellt sich das Problem, dass der Draht besonders an seinem distalen Ende einerseits möglichst flexibel sein soll, um ein möglichst atraumatisches Einführen des Drahtes in Herzgefäße und dabei ein leichtgängiges Passieren von Engstellen und Biegungen in dem anzufahrenden Gefäßverlauf zu gewährleisten, andererseits benötigen die zur Erkennung des Führungsdrahtes bei einer Röntgenüberwachung während der Durchführung einer PTCA verwendeten Marker ein bestimmtes Materialvolumen, um den erreichbaren Kontrast im Röntgenbild auf einem praxisgerechten Niveau zu halten. Die eingangs erwähnten Marker auf Basis eines schraubenförmig gewickelten Drahtwendels sind also auf einen möglichst großen Wickelkern aufzubringen, was der Forderung noch einen möglichst dünnen Durchmesser des Drahtschaftes diametral entgegenläuft.

Zur Lösung dieser Problematik schlägt die Erfindung laut Kennzeichnungsteil des Anspruches 1 vor, dass der oder die Marker jeweils auf einem im Durchmesser gegenüber den angrenzenden Schaft-Abschnitten des Drahtschaftes vergrößerten Kernabschnitt sitzen. Mit anderen Worten wird nur an den Stellen, wo der Marker sitzen soll, eine Positionierfläche zur Verfügung gestellt, die einen gegenüber dem verbleibenden Drahtschaft größeren Durchmesser der Markerhülse erlaubt. Zur Beibehaltung der Flexibilität des Führungsdrahtes sind die zwischen den Markern gelegenen Abschnitte dünner ausgebildet. Die verdickten Kernabschnitte bedingen dabei keine signifikante Verschlechterung des Biegeverhaltens solcher Führungsdrähte, da in den Bereichen der Marker die Flexibilität ohnehin durch diese zusätzliche Komponente eingeschränkt ist. Ferner ist durch den verdickten Kernabschnitt die Wendel besser anlöt- und zentrierbar.

Gemäß bevorzugten Ausführungsformen der Erfindung gehen die Kernabschnitte jeweils beiderseits in rampenartigen Ringschultern in den angrenzenden Schaftabschnitt über. Damit werden scharfe Innenecken und Kanten vermieden, die bei den kleinen Dimensionen solcher Führungsdrähte produktionstechnisch schwierig zu beherrschen sind. Dies trifft insbesondere auf die Bearbeitungsschritte des Verschleifens und Polierens des Drahtes zu. Schließlich würden scharfe Innenecken zu einer erhöhten Kerbbruch-Anfälligkeit des Drahtschaftes führen.

Gemäß einer weiteren bevorzugten Ausführungsform ist es vorgesehen, den als Drahtwendel ausgebildeten Marker jeweils mit einem Lötbett auf dem Kernabschnitt zu fixieren. Dabei läuft das Lötbett an seinen beiden Enden jeweils in einer kegelstumpfförmigen Rampe zum angrenzenden Schaftabschnitt aus, ist also insoweit an die konische Ringschulter der Kernabschnitte angepasst. Insoweit ergeben sich insbesondere nach dem Verschleifen und Polieren eines derartigen Führungsdrahtes mit Marker keine störenden Kanten am Produkt mehr, die Verletzungen der Gefäßwand - sogenannte "Dissektionen - beim Einführen des Drahtes hervorrufen könnten.

Weitere bevorzugte Ausführungsformen betreffen die Dimensionsverhältnisse des Drahtschaftes und mehrerer Marker. Von Vorteil ist hier besonders der etwas geringere Außendurchmesser der Marker im Vergleich mit der üblicher Weise bei solchen Katheterdrähten vorhandenen distalen Markerwendel. Dadurch wird ein ruckartiges Einsetzen von Reibung beim Verschieben des Führungsdrahtes relativ zum PTCA-Katheter vermieden.

Weitere Merkmale, Vorteile und Einzelheiten der Erfindung ergeben sich aus der nachfolgenden Beschreibung, in der ein Ausführungsbeispiel anhand der beigefügten Zeichnungen näher erläutert wird. Es zeigen:
Fig. 1 einen Längsaxialschnitt eines Katheter-Führungsdrahtes, und
Fig. 2 einen vergrößerten Längsaxialabschnitt des distalen Endabschnittes des Führungsdrahtes gemäß Fig. 1.

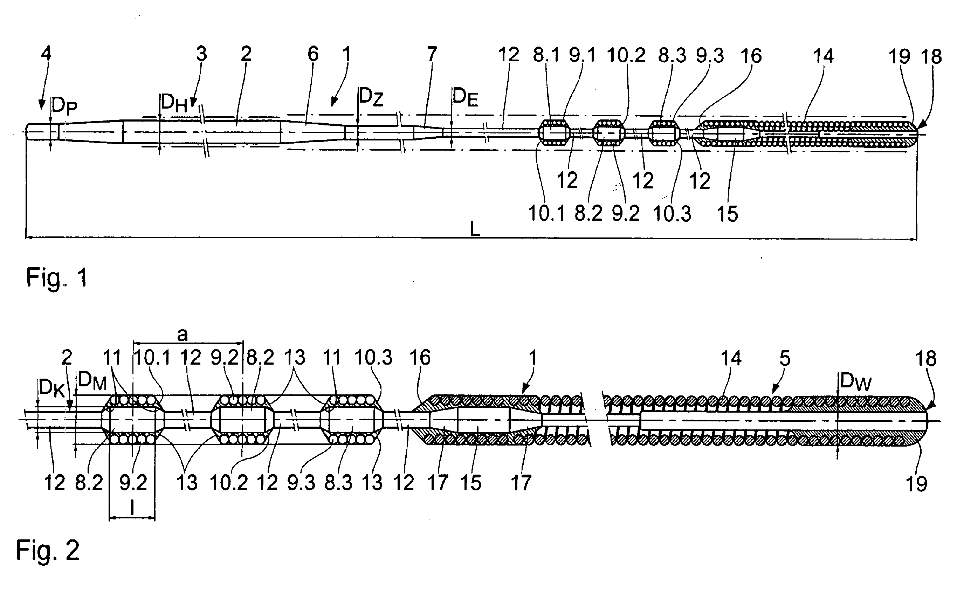

Wie aus Figur 1 hervorgeht, weist der als Ganzes mit 1 bezeichnete Führungsdraht einen langgestreckten Drahtschaft 2 aus medizinischem Edelstahl auf. Über seine Gesamtlänge L von beispielsweise 1750 mm sind Schaftabschnitte mit unterschiedlichen Durchmessern vorgesehen. So hat der Drahtschaft 2 an seinem proximalen Ende 3 einen konisch verjüngten Endabschnitt, dessen Außendurchmesser D_{P} mit beispielsweise 0,223 mm deutlich geringer als der Hauptdurchmesser D_{H} des Drahtschaftes 2 mit 0,36 mm ist. Das konisch verjüngte proximale Ende 3 des Drahtschaftes 2 dient zum Ansetzen einer Drahtverlängerung.

In der vor dem distalen Ende 5 des Führungsdrahtes 5 gelegenen distalen Endzone, die eine Länge von insgesamt etwa 300 mm aufweisen kann, ist der Drahtschaft 2 von seinem Hauptdurchmesser D_{H} über konische Rampen 6, 7 auf einen Zwischendurchmesser D_{Z} von etwa 0,2 mm und auf einen End-Durchmesser D_{E} von beispielsweise 0,12 mm in seiner Stärke reduziert. Dabei werden allerdings an drei Positionen, die einen MittenAbstand a von ca. 10 mm aufweisen, beim Einschleifen im Durchmesser D_{K} vergrößerte Kernabschnitte 8.1., 8. 2, 8.3 gebildet, die als Auflager für jeweils einen radioopaquen Marker 9.1, 9.2, 9.3 dienen. Diese Marker 9 weisen eine Länge 1 von etwa 1 mm auf und sind jeweils durch eine Drahtwendel aus einer Platin-Iridium-Legierung gebildet. Andere Materialien für solche Röntgen-Marker-Wendeln können beispielsweise Gold, Tantal oder Wolfram sein. Der Außendurchmesser D_{M} der Marker 9.1, 9.2, 9.3 entspricht dem Durchmesser D_{H} des Drahtschaftes 2.

Jede der drei Drahtwendeln 9.1, 9.2, 9.3 ist mit einem Lötbett 10.1, 10.2, 10.3 auf dem jeweiligen Kernabschnitt 8.1, 8.2, 8.3 befestigt. Dabei gehen die Kernabschnitte 8.1, 8.2, 8.3 jeweils über konisch ausgebildete, rampenartige Ringschultern 11 in die angrenzenden Schaftabschnitte 12 über. Genauso sind die Lötbetten 10.1, 10.2, 10.3 derart verschliffen und poliert, dass sie mit einer kegelstumpfförmigen Rampe 13 zum jeweiligen Schaftabschnitt 12 hin auslaufen. Die Ringschulter 11 der Kernabschnitte 8.1, 8.2, 8.3 und die konischen Rampen 13 der Lötbetten 10.1, 10.2, 10.3 fluchten in längsaxialer Richtung miteinander.

Am distalen Ende 5 schließlich ist eine sich etwa über 28 mm erstreckende Markerwendel 14 vorgesehen, die an ihrem proximalen Ende auf einer Schaftverdickung 15 mit Hilfe eines mehrere Wendelgänge übergreifenden Lötbetts 16 verbunden ist. Letzteres ist in proximaler Richtung zu dem dünnen Schaftabschnitt 12 hin ebenfalls als konisch verlaufende Rampe 17 ausgeformt. Die Schaftverdickung 15 mit Lötbett 16 zur Fixierung der Markerwendel 14 dient als Übertragungsmittel zur zuverlässigen Einleitung eines Drehmomentes in das distale Ende des Führungsdrahtes 1. An der Spitze 18 des Führungsdrahtes 1 ist die Markerwendel 14 mit Hilfe eines weiteren Lötbetts 19 mit dem dort abgeplatteten Drahtschaft 2 fest verbunden.

Der Außendurchmesser D_{M} der Marker 9.1 ist gleich oder geringfügig kleiner als der Außendurchmesser D_{W} der Markerwendel 14. Der gesamte Führungsdraht 1 ist im Übrigen in bekannter Weise mit von Drahtabschnitt zu Drahtabschnitt wechselnden reibungsvermindernden Beschichtungen beispielsweise auf PTFE- oder Silicon-Basis oder mit einer hydrophilen Materialauflage hauchdünn beschichtet.

## Patentansprüche

1. Katheter-Führungsdraht insbesondere für die perkutane transluminale Coronar-Angioplastik umfassend
- einen langgestreckten Drahtschaft (2) aus einem federelastischen Material, insbesondere aus Edelstahl mit einem proximalen und distalen Ende (3, 5),
- mindestens einen auf dem Drahtschaft (2) sitzenden, radiopaquen, hülsenartigen Marker (9) vor dem distalen Ende (3),
**dadurch gekennzeichnet, dass**
- der mindestens eine Marker (9) auf einem im Durchmesser (D_{K}) gegenüber dem Durchmesser (D_{E}) der angrenzenden Schaft-Abschnitte (12) vergrößerten Kernabschnitt (8) des Drahtschaftes (2) sitzt.

2. Katheter-Führungsdraht nach Anspruch 1, **dadurch gekennzeichnet, dass** der Kernabschnitt (8) jeweils beiderseits in einer konisch ausgebildeten, rampenartigen Ringschulter (11) in den angrenzenden Schaftabschnitt (12) übergeht.

3. Katheter-Führungsdraht nach Anspruch 1 oder 2, **dadurch gekennzeichnet, dass** die Durchmesser-vergrößerten Kernabschnitte (8) für jeden Marker (9) durch Abschleifen des Drahtschaft (2) im Bereich um beiderseits jeweiliges Kernabschnitts (8) gebildet sind.

4. Katheter-Führungsdraht nach einem der vorgenannten Ansprüche, wobei der mindestens eine Marker (9) als auf dem jeweiligen Kernabschnitt (8) sitzende Drahtwendel ausgebildet ist, **dadurch gekennzeichnet, dass** die jeweilige Drahtwendel mit einem Lötbett (10) auf dem Kernabschnitt (8) fixiert ist, das an seinen beiden Enden jeweils in einer kegelstumpfförmigen Rampe (13) zu dem angrenzenden, Durchmesser-verringerten Schaftabschnitt (12) hin ausläuft.

5. Katheter-Führungsdraht nach Anspruch 2 und 4, **dadurch gekennzeichnet, dass** die Ringschulter (11) eines jeweiligen Kernabschnitte (8) und die konische Rampe (13) eines jeweiligen Lötbetts (10) in längsaxialer Richtung miteinander fluchten.

6. Katheter-Führungsdraht nach einem der vorgenannten Ansprüche, **dadurch gekennzeichnet, dass** vor dem mit einer langgestreckten Markerwendel (14) versehenen distalen Ende (5) des Drahtschaftes (2) drei Marker (9) mit Abstand (a) zueinander angeordnet sind.

7. Katheter-Führungsdraht nach Anspruch 6, **dadurch gekennzeichnet, dass** das Verhältnis von Länge (1) jedes Markers (9) zum ihrem Abstand (a) zueinander etwa 1:10 beträgt.

8. Katheter-Führungsdraht nach einem der vorgenannten Ansprüche, **dadurch gekennzeichnet, dass** der Außendurchmesser (D_{M}) des mindestens einen Markers (9) geringfügig kleiner ist als der Außendurchmesser (D_{W}) einer langgestreckten Markerwendel (14) am distalen Ende (5) des Drahtschaftes (2).

9. Katheter-Führungsdraht nach einem der vorgenannten Ansprüche, **dadurch gekennzeichnet, dass** das die Oberfläche des Führungsdrahtes (1) mit seinem Drahtschaft (2) und dem mindestens einen Marker (9) mit Lötbett (10) geglättet, insbesondere verschliffen und poliert ist.

10. Katheter-Führungsdraht nach einem der vorgenannten Ansprüche, **gekennzeichnet durch** eine reibungsvermindernde Beschichtung auf dem Führungsdraht (1).

## Claims

1. A catheter guide wire, in particular for percutaneous transluminal coronary angioplasty, comprising
- an elongated wire shaft (2) of an elastic material, in particular stainless steel, having a proximal end and a distal end (3, 5),
- at least one radiopaque sleeve-like marker (9) sitting on the wire shaft (2) upstream from the distal end (3),
**characterized in that**
- the at least one marker (9) sits on a core section (8) of the wire shaft (2) that is enlarged in diameter (D_{K}) in comparison with the diameter (D_{E}) of the adjacent shaft sections (12).

2. The catheter guide wire according to Claim 1, **characterized in that** the core section (8) develops on both ends into the adjacent shaft section (12) along a ramp-like ring shoulder (11) designed in the form of a conical ramp-like ring shoulder (11).

3. The catheter guide wire according to Claim 1 or 2, **characterized in that** the diameter-enlarged core sections (8) for each marker (9) are formed by grinding the wire shaft (2) in the area around the respective core section (8) on both ends.

4. The catheter guide wire according to any one of the preceding claims, wherein the at least one marker (9) is designed as a wire coil sitting on the respective core section (8), **characterized in that** the respective wire coil is secured with a solder bump (10) on the core section (8), which tapers to the adjacent shaft section (12) with a reduced diameter on each of its two ends in a ramp (13) in the form of a truncated cone.

5. The catheter guide wire according to Claims 2 and 4, **characterized in that** the ring shoulder (11) of a respective core section (8) and the conical ramp (13) of a respective solder bump (10) are aligned with one another in the longitudinal axial direction.

6. The catheter guide wire according to any one of the preceding claims, **characterized in that** three markers (9) are arranged with a distance (a) from one another upstream from the distal end (5) of the wire shaft (2) provided with an elongated marker coil (14).

7. The catheter guide wire according to Claim 6, **characterized in that** the ratio of length (I) of each marker (9) to the distance (a) to one another is approximately 1:10.

8. The catheter guide wire according to any one of the preceding claims, **characterized in that** the outside diameter (D_{M}) of the at least one marker (9) is slightly smaller than the outside diameter (D_{W}) of an elongated marker coil (14) on the distal end (5) of the wire shaft (2).

9. The catheter guide wire according to any one of the preceding claims, **characterized in that** the surface of the guide wire (1) is smoothed with a solder bump (10), in particular is ground and polished with its wire shaft (2) and the at least one marker (9).

10. The catheter guide wire according to any one of the aforementioned claims, **characterized by** a friction-reducing coating on the guide wire (1).

## Revendications

1. Fil de guidage à cathéter en particulier pour l'angioplastie transluminale coronaire percutanée comprenant :
- une tige de fil (2) étirée en longueur à base d'un matériau élastique, en particulier à base d'acier fin avec une extrémité proximale et une proximité distale (3, 5),
- au moins un marqueur (9) logé sur la tige de fil (2), radio-opaque, de type douille, avant l'extrémité (3) distale, **caractérisé en ce que**
- le au moins un marqueur (9) est logé sur une partie centrale (8), agrandie en diamètre (D_{K}) par rapport au diamètre (D_{E}) des parties de tige (12) contiguës, de la tige de fil (2).

2. Fil de guidage à cathéter selon la revendication 1, **caractérisé en ce que** la partie centrale (8) fait place à la partie de tige (12) contiguë à chaque fois des deux côtés dans un épaulement annulaire (11) conçu en cône et de type rampe.

3. Fil de guidage à cathéter selon la revendication 1 ou 2, **caractérisé en ce que** les parties centrales (8) agrandies en diamètre pour chaque marqueur (9) sont formées par rectification de la tige de fil (2) dans la zone située autour des deux côtés de la partie centrale (8) respective.

4. Fil de guidage à cathéter selon l'une quelconque des revendications précédentes, le au moins un marqueur (9) étant réalisé sous la forme d'un filament de fil logé sur la partie centrale (8) respective, **caractérisé en ce que** le filament de fil respectif est fixé avec un lit de brasage (10) sur la partie centrale (8), qui se termine sur ses deux extrémités respectivement dans une rampe (13) en forme de cône tronqué, en direction de la partie de tige (12) contiguë et réduit en diamètre.

5. Fil de guidage à cathéter selon les revendications 2 et 4, **caractérisé en ce que** l'épaulement annulaire (11) d'une partie centrale (8) respective et la rampe (13) conique d'un lit de brasage (10) respectif sont alignées l'une avec l'autre dans la direction axiale longitudinale.

6. Fil de guidage à cathéter selon l'une quelconque des revendications précédentes, **caractérisé en ce que** trois marqueurs (9) sont disposés à la distance (a) les uns des autres avant l'extrémité (5) distale, dotée d'un filament de marqueur (14) étiré en longueur, de la tige de fil (2).

7. Fil de guidage à cathéter selon la revendication 6, **caractérisé en ce que** le rapport entre la longueur (1) de chaque marqueur (9) et son espacement (a) est d'environ 1:10.

8. Fil de guidage à cathéter selon l'une quelconque des revendications précédentes, **caractérisé en ce que** le diamètre extérieur (D_{M}) du au moins un marqueur (9) est légèrement inférieur au diamètre extérieur (D_{W}) d'un filament de marqueur (14) étiré en longueur sur l'extrémité (5) distale de la tige de fil (2).

9. Fil de guidage à cathéter selon l'une quelconque des revendications précédentes, **caractérisé en ce que** la surface du fil de guidage (1) est lissée, en particulier poncée et polie avec sa tige de fil (2) et le au moins un marqueur (9) avec le lit de brasage (10).

10. Fil de guidage à cathéter selon l'une quelconque des revendications précédentes **caractérisé par** un revêtement réduisant le frottement sur le fil de guidage (1).
